# EUROPEAN PATENT APPLICATION

(11) **EP 4 410 981 A1**
(43) Date of publication of application: **07.08.2024**
(21) Application number: 22876181.3
(22) Date of filing: 27.09.2022
(51) Int. Cl.: C12N 15/57, A23L 13/70, C12N 9/52

(54) **ENZYME AGENT FOR COLLAGEN TRIPEPTIDE PRODUCTION OR CARTILAGE DEGRADATION**

(30) Priority: 28.09.2021 JP 2021157337
(71) Applicant: Amano Enzyme Inc., Nagoya-shi Aichi 460-8630 (JP)
(72) Inventor: KAMON, Masahiro, Kitanagoya-shi, Aichi 481-8533 (JP); YACHI, Hiroyuki, Kakamigahara-shi, Gifu 509-0109 (JP); ISHIGAKI, Yuki, Kakamigahara-shi, Gifu 509-0109 (JP)
(74) Representative: Witthoff Jaekel Steinecke Patentanwälte PartG mbB
(86) International application number: PCT/JP2022/035843
(87) International publication number: WO 2023/054315

(57) **Abstract**

It is an object of the present invention to provide a highly safe protease (collagenase) that is useful for food or medical applications, such as collagen tripeptide production or cartilage degradation, the intended use thereof, and the like. According to the present invention, provided is an enzyme agent for collagen tripeptide production, comprising, as an active ingredient, a collagenase consisting of the amino acid sequence as set forth in SEQ ID NO: 1 or an amino acid sequence equivalent thereto.

## Description

### Technical Field

The present invention relates to an enzyme agent for collagen tripeptide production or cartilage degradation, comprising a collagenase as an active ingredient, and the intended use thereof.

### Background Art

The global market of collagen peptides known as functional peptides has been predicted to grow. Among such collagen peptides, the tripeptide Gly-X-Y that is a minimum unit of collagen (i.e. collagen tripeptide; hereinafter also abbreviated as "CTP") is highly absorbed into the body and also has various functionalities. Thus, the use of CTP is considered to be highly valuable.

In order to efficiently produce CTP, protease (collagenase) capable of specifically cleaving collagen or gelatin at the position of a Gly residue and decomposing it even to a tripeptide is useful. Examples of the known collagenase may include collagenase derived from Clostridium sp. or Vibrio sp., and *Bacillus cereus* collagenase (belonging to Microbial collagenase (EC.3.4.24.3)). However, the collagenase-producing bacteria have biosafety level 2 (BSL2), and thus, the safety thereof has been concerned. Accordingly, it is said that the use of the collagenase-producing bacteria is not suitable for the production of CTP that is utilized for food use, medical use, etc.

As a collagenase usable for food use, a collagenase derived from Streptomyces sp. has been known (Patent Document 1). However, whether this collagenase can be utilized in the production of CTP has not been known. In addition, the existing collagenases are generally problematic in terms of stability. As a microorganism-derived collagenase having excellent stability and high specific activity, *Vibrio hollisae-derived* collagenase (Vibrio sp. 1706B strain-derived collagenase disclosed in Patent Document 2) has been known (Patent Document 2 and Patent Document 3). However, the heat stability of the *Vibrio hollisae-derived* collagenase is 30°C or lower, and it is practically insufficient.

Moreover, it has been known that various collagenases having different properties are present. For example, it has been known that *Clostridium histolyticum-*derived collagenase has two different collagenase types (I and II), and that collagenase I has higher activity on collagen and gelatin and lower activity on a short-chain peptide, than collagenase II does (Patent Document 4). Furthermore, it has also been known that *Clostridium histolyticum-*derived collagenase has a low decomposition rate of cleaving CTP from a collagen-like sequence (Patent Document 5).

Collagenase suitable for producing CTP usable for food use from collagen is required to be produced from safe collagenase-producing bacteria, to have collagen-decomposing ability or gelatin-decomposing ability, and to have CTP-producing ability. Preferably, such collagenase is also required to have high safety. However, collagenase that satisfies the aforementioned conditions and has been put to practical use has not yet been known.

On the other hand, there is a case where collagenase having high cartilage degradation ability is demanded for food applications. However, such collagenase having high cartilage degradation ability has not yet been known.

### Prior Art Documents

### Patent Documents

Patent Document 1: JP Patent Publication No. 5-16832 B (1993)
Patent Document 2: JP Patent Publication No. 8-70853 A (1996)
Patent Document 3: JP Patent Publication No. 2010-263880 A
Patent Document 4: JP Patent Publication No. 2001-510331 A
Patent Document 5: JP Patent Publication No. 2018-183106 A

### Summary of Invention

### Objects to be Solved by the Invention

Under the above-described background, it is an object of the present invention to provide: a highly safe protease (collagenase) that is useful for food or medical applications, such as collagen tripeptide production or cartilage degradation; the intended use thereof; etc.

### Means for Solving the Objects

The present inventors have screened a wide variety of microorganism-derived enzymes directed towards obtaining a collagenase having high CTP-producing ability, high cartilage degradation ability, and high safety. As a result, it was revealed that a specific strain of *Lysinibacillus fusiformis* produces collagenase that corresponds to the purpose. This collagenase exhibited collagenase-decomposing ability and CTP-producing ability, and further, this collagenase had high cartilage degradation ability, thereby being industrially highly valuable. According to the present invention, the following inventions are provided.

< 1 > An enzyme agent for collagen tripeptide production, including, as an active ingredient, a collagenase consisting of the amino acid sequence as set forth in SEQ ID NO: 1 or an amino acid sequence equivalent thereto.
< 2 > The enzyme agent according to < 1 >, in which the collagenase consisting of the equivalent amino acid sequence has an identity of 90% or more to the amino acid sequence as set forth in SEQ ID NO: 1, and has an ability to produce Gly-Glu-Arg from beef bone gelatin that is equivalent to that of the collagenase consisting of the amino acid sequence as set forth in SEQ ID NO: 1.
< 3 > The enzyme agent according to < 1 > or < 2 >, in which the collagenase is derived from *Lysinibacillus fusiformis.*
< 4 > An enzyme agent for collagen tripeptide production, which is derived from *Lysinibacillus fusiformis* and includes, as an active ingredient, a collagenase having the following physicochemical properties:
   (1) optimal temperature: around 40°C;
   (2) temperature stability: relative residual activity to the activity at a treatment temperature with the highest activity as 100% is 80% or more from 4°C to 40°C, and is 10% or less at 50°C;
   (3) optimal pH: around pH 7; and
   (4) pH stability: relative residual activity to the activity at the treatment pH with the highest activity as 100% is 80% or more in the pH range of 6 to 8, and is 10% or less at pH 5.
< 5 > An enzyme agent for edible meat tenderization and/or cartilage degradation, including, as an active ingredient, a collagenase consisting of the amino acid sequence as set forth in SEQ ID NO: 1 or an amino acid sequence equivalent thereto.
< 6 > The enzyme agent according to < 5 >, in which the collagenase consisting of the equivalent amino acid sequence has an identity of 90% or more to the amino acid sequence as set forth in SEQ ID NO: 1, and has a chicken cartilage degradation ability equivalent to that of the collagenase consisting of the amino acid sequence as set forth in SEQ ID NO: 1.
< 7 > The enzyme agent according to < 5 > or < 6 >, in which the collagenase is derived from *Lysinibacillus fusiformis.*
< 8 > An enzyme agent for edible meat tenderization and/or cartilage degradation, which is derived from *Lysinibacillus fusiformis* and includes, as an active ingredient, a collagenase having the following physicochemical properties:
   (1) optimal temperature: around 40°C;
   (2) temperature stability: relative residual activity to the activity at a treatment temperature with the highest activity as 100% is 80% or more from 4°C to 40°C, and is 10% or less at 50°C;
   (3) optimal pH: around pH 7; and
   (4) pH stability: relative residual activity to the activity at the treatment pH with the highest activity as 100% is 80% or more in the pH range of 6 to 8, and is 10% or less at pH 5.
< 9 > A method for producing a collagen tripeptide, which is characterized in that it includes allowing the enzyme agent according to any one of < 1 > to < 4 > to act on collagen or gelatin.
< 10 > A method for edible meat tenderization and/or cartilage degradation, which is characterized in that it includes allowing the enzyme agent according to any one of < 5 > to < 8 > to act on edible meat and/or cartilage.

### Brief Description of Drawings

[Fig. 1] Fig. 1 shows the optimal temperature of the collagenase derived from the *Lysinibacillus fusiformis* 7128 strain.
[Fig. 2] Fig. 2 shows the temperature stability of the collagenase derived from the *Lysinibacillus fusiformis* 7128 strain.
[Fig. 3] Fig. 3 shows the optimal pH of the collagenase derived from the *Lysinibacillus fusiformis* 7128 strain.
[Fig. 4] Fig. 4 shows the pH stability of the collagenase derived from the *Lysinibacillus fusiformis* 7128 strain.
[Fig. 5] Fig. 5 shows the results obtained by confirming the ability of the enzyme to produce the collagen tripeptide Gly-Pro-Hyp (fish gelatin).
[Fig. 6] Fig. 6 shows the results obtained by confirming the ability of the enzyme to produce the collagen tripeptide Gly-Pro-Ala (fish gelatin).
[Fig. 7] Fig. 7 shows the results obtained by confirming the ability of the enzyme to produce the collagen tripeptide Gly-Glu-Arg (fish gelatin).
[Fig. 8] Fig. 8 shows the results obtained by confirming the ability of the enzyme to produce the collagen tripeptide Gly-Glu-Arg (beef bone gelatin).
[Fig. 9] Fig. 9 shows the results obtained by measuring a cartilage degradation ability.

### Embodiments of Carrying out the Invention

### 1. Enzyme agent for collagen tripeptide production or cartilage degradation

A first aspect of the present invention relates to an enzyme agent for collagen tripeptide production or edible meat tenderization and/or cartilage degradation. The enzyme agent of the present invention (hereinafter also referred to as "the present enzyme agent") includes, as an active ingredient, a collagenase (hereinafter also referred to as "the present enzyme"). The enzyme agent of the present invention is useful for collagen tripeptide production or edible meat tenderization and/or cartilage degradation (the details will be described later). The collagenase serving as an active ingredient, namely, the present enzyme consists of the amino acid sequence as set forth in SEQ ID NO: 1, or an amino acid sequence equivalent to the amino acid sequence as set forth in SEQ ID NO: 1. Herein, the term "equivalent amino acid sequence" means an amino acid sequence that is partially different from the reference amino acid sequence (i.e. the amino acid sequence as set forth in SEQ ID NO: 1) but such difference does not substantially influence on the function of the protein (which is herein a collagen-decomposing activity), and in the present invention, the term "equivalent amino acid sequence" further means an amino acid sequence having an equivalent ability to produce Gly-Glu-Arg from beef bone gelatin or an amino acid sequence having an equivalent chicken cartilage degradation ability. Accordingly, an enzyme having such an equivalent amino acid sequence catalyzes a collagen-decomposing reaction, and has an ability to produce Gly-Glu-Arg from beef bone gelatin and/or a chicken cartilage degradation ability. The degree of each of the above-described collagen-decomposing activity, ability to produce Gly-Glu-Arg, and chicken cartilage degradation ability is not particularly limited, as long as the function of a collagenase can be exhibited. However, the activity of the enzyme having such an equivalent amino acid sequence is preferably equivalent to or higher than the activity of an enzyme consisting of the reference amino acid sequence (i.e. an enzyme having the amino acid sequence as set forth in SEQ ID NO: 1). The phrase "an equivalent ability to produce Gly-Glu-Arg from beef bone gelatin" means that the present enzyme has a producing ability that is ±10%, and preferably ±5%, compared with the collagenase having the reference amino acid sequence. The phrase "an equivalent chicken cartilage degradation ability" means that the present enzyme has a degradation ability that is ±10%, and preferably ±5%, compared with the collagenase having the reference amino acid sequence.

The amino acid sequence as set forth in SEQ ID NO: 1 is the amino acid sequence of the collagenase derived from *Lysinibacillus fusiformis* (mature body). Besides, the amino acid sequence of the collagenase derived from *Lysinibacillus fusiformis,* which also has a signal peptide and a pro-sequence, is shown in SEQ ID NO: 2.

A "partial difference in an amino acid sequence" is produced, for example, as a result of a deletion or a substitution of one or more amino acids in the amino acids constituting the amino acid sequence, or an addition or an insertion of one or more amino acids into the amino acid sequence, or any given combination thereof. Such a partial difference in the amino acid sequence is acceptable, as long as the collagen-decomposing activity, the ability to produce Gly-Glu-Arg, and/or the chicken cartilage degradation ability are retained (in which the activity may be slightly fluctuated). As far as this condition is satisfies, the position of the amino acid sequence, in which the difference is found, is not particularly limited. In addition, such difference may be produced in multiple sites (places) on the amino acid sequence.

The number of amino acids providing such a partial difference in the amino acid sequence is a number corresponding to, for example, about 20% or less, preferably about 15% or less, more preferably about 10% or less, even more preferably about 5% or less, further preferably about 3% or less, even further preferably about 2% or less, still further preferably about 1% or less, still further preferably about 0.7% or less, still further preferably about 0.5% or less, still further preferably about 0.3% or less, and still further preferably about 0.1% or less, with respect to the entire amino acids that constitute the amino acid sequence. Accordingly, the equivalent protein has an identity of, for example, about 80% or more, preferably about 85% or more, more preferably about 90% or more, even more preferably about 95% or more, further preferably about 97% or more, even further preferably about 98% or more, still further preferably about 99% or more, still further preferably about 99.3% or more, still further preferably about 99.5% or more, still further preferably about 99.7% or more, and still further preferably about 99.9% or more, to the reference amino acid sequence.

A typical example of a "partial difference in the amino acid sequence" is that a mutation (a change) is produced in the amino acid sequence, as a result of a deletion or a substitution of 1 to 40 (preferably 1 to 30, more preferably 1 to 10, even more preferably 1 to 7, further preferably 1 to 5, and still further preferably 1 to 3) amino acids in the amino acids constituting the amino acid sequence, or an addition or an insertion of 1 to 40 (preferably 1 to 30, more preferably 1 to 10, even more preferably 1 to 7, further preferably 1 to 5, and still further preferably 1 to 3) amino acids into an amino acid sequence, or any given combination thereof.

Preferably, an equivalent amino acid sequence is obtained by the occurrence of conservative amino acid substitution in amino acid residues that are not essential for collagen-decomposing ability. The term "conservative amino acid substitution" is used herein to mean that a certain amino acid residue is substituted with an amino acid residue having a side chain with similar properties. Amino acid residues are classified into several families, depending on the side chain thereof; namely, basic side chains (for example, lysine, arginine, and histidine), acidic side chains (for example, aspartic acid and glutamic acid), uncharged polar side chains (for example, glycine, asparagine, glutamine, serine, threonine, tyrosine, and cysteine), non-polar side chains (for example, alanine, valine, leucine, isoleucine, proline, phenylalanine, methionine, and tryptophan), β-branched side chains (for example, threonine, valine, and isoleucine), aromatic side chains (for example, tyrosine, phenylalanine, tryptophan, and histidine), etc. The conservative amino acid substitution is preferably a substitution occurring between amino acid residues in an identical family.

The identity (%) of two amino acid sequences can be determined, for example, by the following procedures. First, two sequences are aligned such that an optimal comparison can be made (for example, a gap may be introduced into a first sequence, so that the alignment with a second sequence may be optimized). When a molecule (amino acid residue) in a specific position of the first sequence is identical to a molecule in a corresponding position in the second sequence, it can be said that the molecules at the positions are identical to each other. The identity of the two sequences is a function of the number of identical positions common in the two sequences (i.e. identity (%) = the number of identical positions / total number of positions x 100), and preferably, the number and size of gaps used for optimization of the alignment are also taken into consideration.

Comparison of two sequences and determination of identity can be realized using mathematical algorithms. A specific example of the mathematical algorithm that can be utilized in comparison of sequences may be an algorithm that is described in Karlin and Altschul (1990) Proc. Natl. Acad. Sci. USA 87: 2264-68 and is modified in Karlin and Altschul (1993) Proc. Natl. Acad. Sci. USA 90: 5873-77, but the example of the mathematical algorithm is not limited thereto. The identity of amino acid sequences can be obtained, for example, by blastp (protein-protein BLAST) of National Center for Biotechnology Information (NCBI). As parameters, default parameters may be used. For example, using BLOSUM62 matrix, Gap Costs may be set to be Existence: 11 and Extension: 1.

The collagenase that is an active ingredient of the present enzyme agent, namely, the present enzyme may be a part of a larger protein (for example, a fusion protein). Examples of a sequence to be added to such a fusion protein may include sequences that are useful for purification, such as multiple histidine residues, and additional sequences that ensure stability during recombinant production.

The present enzyme can be obtained by culturing microorganisms that produce the present collagenase (i.e. a collagenase-producing strain), for example, *Lysinibacillus fusiformis.* The collagenase-producing strain may be either a wild-type strain or a mutant strain (such a mutant strain is obtained, for example, by ultraviolet irradiation).

The present enzyme can be prepared using a culture solution and/or a cell mass of microorganisms that produce the present enzyme. The culture conditions or the culture methods are not particularly limited, as long as the present enzyme can be produced thereby. That is to say, the methods or the culture conditions that are adapted to the culture of the used microorganisms can be determined, as appropriate, under conditions in which the present enzyme is produced. Regarding the culture method, either liquid culture or solid culture may be applied, and liquid culture is preferably utilized. Taking the liquid culture as an example, the culture conditions will be described below.

The medium is not particularly limited, as long as it is a medium in which the used microorganisms can grow. Examples of the medium that can be used herein may include those to which the following substances are added: carbon sources, such as glucose, sucrose, gentiobiose, soluble starch, glycerin, dextrin, molasses, and organic acids; nitrogen sources, such as ammonium sulfate, ammonium carbonate, ammonium phosphate, ammonium acetate, or gelatin, peptone, yeast extract, corn steep liquor, casein hydrolysate, bran, and meat extract; and further, inorganic salts, such as potassium salts, magnesium salts, sodium salts, phosphates, manganese salts, iron salts and zinc salts. In order to promote the growth of the used microorganisms, vitamins, amino acids and the like may be added to the medium. The pH of the medium is adjusted to, for example, about 3 to 8, preferably about 4 to 7. The culture temperature is usually about 20°C to 40°C, preferably about 25°C to 35°C, and the microorganisms are cultured for 1 to 20 days, and preferably 3 to 10 days, under aerobic conditions. As a culture method applied herein, for example, a shaking culture method, or an aerobic deep culture method using ajar fermenter, can be utilized.

After completion of the culture performed under the above-described conditions, the enzyme of interest is recovered from the culture solution or the culture mass. When the enzyme is recovered from the culture solution, for example, the culture supernatant is filtered, centrifuged, etc. to remove insoluble matters, and thereafter, the resultant is separated and purified by appropriately combining concentration using an ultrafiltration membrane, salting-out such as ammonium sulfate precipitation, dialysis, and various types of chromatography using ion exchange resin, etc., thereby obtaining the present enzyme. On the other hand, when the enzyme is recovered from the cell mass, for example, the cell mass is crushed by a pressure treatment, an ultrasonic treatment, etc., and is then the resultant is separated and purified in the same manner as that described above, so as to obtain the present enzyme. Besides, the cell mass may be previously recovered from the culture solution by filtration, a centrifugation treatment, etc., and thereafter, the above-described series of steps (crushing of the cell mass, separation, and purification) may be carried out.

Also, the present enzyme can be easily prepared by a genetic engineering technique. For example, the present enzyme can be prepared by transforming suitable host cells (for example, *Escherichia coli*) with DNA encoding the present enzyme, and then recovering the protein expressed in the transformant. The recovered protein is appropriately purified, depending on the purpose. As such, if the enzyme of interest is obtained as a recombinant protein, various modifications can be carried out thereon. For example, a DNA encoding the present enzyme and another appropriate DNA are inserted into an identical vector, and a recombinant protein is produced using the vector, so that the present enzyme consisting of a recombinant protein, in which any given peptides or proteins are ligated to each other, can be obtained. In addition, modification may be carried out, so that addition of a sugar chain and/or a lipid or the processing of the N-terminus or the C-terminus may occur. By performing the aforementioned modification, simplification of the extraction and purification of a recombinant protein, addition of biological functions, etc. can be carried out.

Generally, the expression of a gene and the recovery of a gene expression product (the present enzyme) are carried out, utilizing an appropriate host-vector system, as described above. However, a cell-free synthesis system may also be utilized. Herein, the term "cell-free synthesis system (a cell-free transcription system or a cell-free transcription/translation system)" means that, not using living cells, but using a ribosome, a transcription/translation factor or the like derived from living cells (or obtained by a genetic engineering technique), from a nucleic acid (DNA or mRNA) used as a template, mRNA or a protein encoded by the nucleic acid is synthesized *in vitro.* In general, in the cell-free synthesis system, a cell extract obtained by purifying, as necessary, a cell-disintegrated solution is used. The cell extract generally includes a ribosome, various types of factors such as an initiation factor, and various types of enzymes such as tRNA, which are necessary for protein synthesis. When a protein is synthesized, various types of amino acids, energy sources such as ATP or GTP, and other substances necessary for protein synthesis, such as creatine phosphate, are added to the aforementioned cell extract. As a matter of course, upon the synthesis of a protein, a ribosome, various types of factors, and/or various types of enzymes, and the like, which are prepared separately, may also be added to the aforementioned cell extract, as necessary.

The development of a transcription/translation system, in which various molecules (factors) necessary for protein synthesis have been reconstructed, has also been reported (Shimizu, Y. et al.: Nature Biotech., 19, 751-755, 2001). In this synthesis system, genes of 31 types of factors composed of: 3 types of initiation factors, 3 types of elongation factors, 4 types of factors associated with termination, 20 types of aminoacyl tRNA synthesis enzymes that allow each amino acid to bind to tRNA, and a methionyl tRNA formyl transfer enzyme, which constitute a protein synthesis system of bacteria, are amplified from an *Escherichia coli* genome, and then, using these amplified products, a protein synthesis system is reconstructed *in vitro.* In the present invention, such a reconstructed synthesis system may be utilized.

The term "cell-free transcription/translation system" is exchangeably used with the term "cell-free protein synthesis system," *"in vitro* translation system" or *"in vitro* transcription/translation system." In the *in vitro* translation system, RNA is used as a template to synthesize a protein. As such template RNA, total RNA, mRNA, an *in vitro* transcriptional product or the like is used. On the other hand, in the *in vitro* transcription/translation system, DNA is used as a template. The template DNA should include a ribosome-binding region, and preferably includes an appropriate terminator sequence. In addition, in the *in vitro* transcription/translation system, in order to promote continuous progression of the transcription reaction and the translation reaction, conditions, in which factors necessary for each reaction are added, are established.

The purified enzyme obtained as described above is pulverized, for example, by freeze drying, vacuum drying, or spray drying, so that the enzyme can be provided in the form of powders. At that time, the purified enzyme may be previously dissolved in an acetate buffer, a phosphate buffer, a triethanolamine buffer, a Tris-HCl buffer, or a GOOD buffer. Preferably, an acetate buffer, a phosphate buffer, or a triethanolamine buffer can be used. Besides, examples of the GOOD buffer used herein may include PIPES, MES, and MOPS.

The purification degree of the enzyme is not particularly limited, and for example, the enzyme can be purified, so that the Pz-peptide-decomposing activity becomes 1 to 20000 (U/g), preferably 10 to 10000 (U/g), and more preferably 100 to 1000 (U/g). In addition, the final form of the enzyme may be either a liquid or a solid (including powders).

As a result of the studies conducted by the present inventors, the properties of the collagenase derived from *Lysinibacillus fusiformis,* consisting of the amino acid sequence as set forth in SEQ ID NO: 1, have been determined as follows (please refer to the after-mentioned Examples for details). Accordingly, the present enzyme can also be specified by the following enzymatic properties. It is to be noted that the details of the measuring conditions, measuring procedures, etc. of collagenase activity necessary for evaluation of each enzymatic property will be described in the after-mentioned Examples.

### (1) Action

The present enzyme is a collagenase, which acts on collagen or gelatin to produce a collagen tripeptide.

### (2) Optimal temperature

The optimal temperature of the present enzyme is 40°C.

### (3) Temperature stability

Even if the present enzyme is treated in a Tris-HCl buffer under conditions of pH 7 and a temperature of 40°C or lower (0°C to 40°C) for 30 minutes, the activity of the enzyme is not substantially decreased. When the present enzyme is treated under the above-described conditions at a temperature of 50°C to 60°C for 30 minutes, the activity is decreased to 10% or less. This is useful in that, upon deactivation of the enzyme, the enzyme can be deactivated at a relatively low temperature in order to prevent excessive progression of the reaction.

### (4) Optimal pH

The optimal pH of the present enzyme is about 7. The optimal pH is determined, for example, based on the results measured in an acetate buffer in the pH range from pH 4 to 6, in a PIPES buffer in the pH range from pH 6 to 7, or in a Tris-HCl buffer (Tris-HCl) in the pH range from pH 7 to 9.

### (5) pH Stability

The present enzyme exhibits stable activity in the pH range from pH 6 to 8. For example, if the pH of an enzyme solution to be treated is within this range, the present enzyme exhibits an activity of 70% or more of the maximum activity, after it has been treated at 30°C for 30 minutes. On the other hand, the activity is decreased to 10% or less of the maximum activity at pH 5. This is useful in that, upon deactivation of the enzyme, the enzyme can be deactivated at a relatively weak acidity in order to prevent excessive progression of the reaction. The pH stability is determined, for example, based on the results measured in an acetate buffer in the pH range from pH 4 to 6, in a PIPES buffer in the pH range from pH 6 to 7, in a Tris-HCl buffer (Tris-HCl) in the pH range from pH 7 to 9, or in a glycine buffer in the pH range from pH 9 to 11.

By allowing the present enzyme to act on collagen or gelatin, there can be obtained the collagen tripeptides Gly-X-Y (CTPs) having Gly (glycine) at the N-terminus, such as Gly-Pro-Hyp, Gly-Pro-Ala and Gly-Glu-Arg. The present enzyme is characterized in that, when the present enzyme is allowed to act on collagen or gelatin, the yield of Gly-Glu-Arg becomes particularly high.

The content of the active ingredient (the present enzyme) in the present enzyme agent is not particularly limited. For example, the content of the active ingredient can be determined or adjusted, so that the Pz-peptide-decomposing activity per gram of the present enzyme agent becomes 1 U to 500 U, and preferably 10 U to 300 U. The enzyme agent of the present invention is usually provided in the form of a solid (for example, a granule, a powder, or an immobilized enzyme formed by immobilizing the present enzyme on a material capable of immobilizing the enzyme on the surface or inside thereof, such as a silica, or a porous polymer) or a liquid. The present enzyme agent may include an excipient, a buffer agent, a suspending agent, a stabilizer, a preservative, an antiseptic, a normal saline, etc., as well as the active ingredient (the present enzyme). As such an excipient, lactose, sorbitol, D-mannitol, maltodextrin, saccharose, etc. can be used. As such a buffer agent, phosphate, citrate, acetate, etc. can be used. As such a stabilizer, propylene glycol, ascorbic acid, etc. can be used. As such a preservative, phenol, benzalkonium chloride, benzyl alcohol, chlorobutanol, methylparaben, etc. can be used. As such an antiseptic, benzalkonium chloride, paraoxybenzoic acid, chlorobutanol, etc. can be used.

### 2. Gene

The present enzyme may be produced by obtaining a gene encoding the present enzyme and then allowing the obtained gene to express. In one aspect, the gene encoding the present enzyme consists of DNA encoding the amino acid sequence as set forth in SEQ ID NO: 1. Specific examples of the present aspect may include DNA consisting of the nucleotide sequence as set forth in SEQ ID NO: 3 and DNA consisting of the nucleotide sequence as set forth in SEQ ID NO: 4. The former DNA (SEQ ID NO: 3) encodes only the amino acid sequence of a mature body (SEQ ID NO: 1), whereas the latter DNA (SEQ ID NO: 4) encodes a signal peptide and a pro-sequence, in addition to the mature body (the amino acid sequence as set forth in SEQ ID NO: 1).

The gene encoding the present enzyme is typically utilized in preparation of the present enzyme. According to a genetic engineering preparation method using the gene encoding the present enzyme, it is possible to obtain the present enzyme in a more homogeneous state. In addition, it is said that this method is a method preferably used in the case of preparing a large amount of the present enzyme. Besides, the intended use of the gene encoding the present enzyme is not limited to the preparation of the present enzyme. For example, the present nucleic acid can also be used as an experimental tool for elucidating the action mechanism of the present enzyme, or as a tool for designing or preparing a mutant (a modified body) of the present enzyme.

In the present description, the phrase "the gene encoding the present enzyme" means a nucleic acid for providing the present enzyme, when the nucleic acid is allowed to express. Thus, the gene encoding the present enzyme includes not only a nucleic acid having a nucleotide sequence corresponding to the amino acid sequence of the present enzyme, but also a nucleic acid formed by adding a sequence that does not encode the amino acid sequence to the aforementioned nucleic acid. Moreover, codon degeneracy is also taken into consideration.

The nucleic acid can be prepared in an isolated state according to a standard genetic engineering technique, a molecular biological technique, a biochemical technique, chemical synthesis, a PCR method (for example, overlap PCR), or a combination thereof, with reference to the present description or the sequence information disclosed in the sequence listing attached herewith.

According to another aspect of the present invention, provided is a nucleic acid, in which when the nucleic acid is compared with the nucleotide sequence of the gene encoding the present enzyme, the function of a protein encoded thereby is equivalent, but the nucleotide sequence thereof is partially different from the nucleotide sequence of the gene encoding the present enzyme (which is hereinafter also referred to as an "equivalent nucleic acid," and the nucleotide sequence of the equivalent nucleic acid is also referred to as an "equivalent nucleotide sequence"). An example of such an equivalent nucleic acid may be DNA that encodes a protein consisting of a nucleotide sequence including a substitution, deletion, insertion, addition or inversion of one or more nucleotides, based on the nucleotide sequence of the nucleic acid encoding the enzyme of the present invention, and having enzyme activity characteristic of the present enzyme (i.e. collagenase activity). Substitution, deletion, etc. of a nucleotide(s) may occur in multiple sites. The term "multiple" is used herein to mean, for example, 2 to 40 nucleotides, preferably 2 to 20 nucleotides, and more preferably 2 to 10 nucleotides, although the number of nucleotides is different depending on the positions or types of amino acid residues in the three-dimensional structure of a protein encoded by the nucleic acid. The equivalent nucleic acid has an identity of, for example, 90% or more, preferably 92% or more, more preferably 94% or more, even more preferably 96% or more, further preferably about 98% or more, and most preferably 99% or more, with respect to the nucleotide sequence serving as a reference (SEQ ID NO: 3 or SEQ ID NO: 4).

Such an equivalent nucleic acid as described above can be obtained by, for example, a restriction enzyme treatment, a treatment with exonuclease, DNA ligase, etc., introduction of a mutation according to a site-directed mutagenesis (Molecular Cloning, Third Edition, Chapter 13, Cold Spring Harbor Laboratory Press, New York) or a random mutation introduction method (Molecular Cloning, Third Edition, Chapter 13, Cold Spring Harbor Laboratory Press, New York), etc.. The equivalent nucleic acid can also be obtained by other methods such as ultraviolet irradiation.

In the present invention, there may be used a nucleic acid having a nucleotide sequence complementary to the nucleotide sequence of the gene of the present invention that encodes the present enzyme. A further aspect of the present invention provides a nucleic acid having a nucleotide sequence that is, at least, about 90%, 92%, 94%, 96%, 98% or 99% identical to the nucleotide sequence of the gene of the present invention that encodes the present enzyme, or to a nucleotide sequence complementary to the nucleotide sequence of the gene of the present invention that encodes the present enzyme.

In the present invention, there may be used a nucleic acid having a nucleotide sequence that hybridizes under stringent conditions to a nucleotide sequence complementary to the nucleotide sequence of the gene of the present invention that encodes the present enzyme or a nucleotide sequence equivalent thereto. The term "stringent conditions" is used herein to mean conditions under which, what is called, a specific hybrid is formed, but a non-specific hybrid is not formed. Such stringent conditions are known to those skilled in the art, and can be determined, for example, with reference to Molecular Cloning (Third Edition, Cold Spring Harbor Laboratory Press, New York) or Current protocols in molecular biology (edited by Frederick M. Ausubel et al., 1987). The stringent conditions may be, for example, conditions in which incubation is performed at about 50°C, using a hybridization solution (50% formamide, 10 x SSC (0.15 M NaCl, 15 mM sodium citrate, pH 7.0), 5 x Denhardt solution, 1% SDS, 10% dextran sulfate, 10 *µ* g/ml denatured salmon sperm DNA, and 50 mM phosphate buffer (pH 7.5)), and thereafter, washing is performed at about 65°C, using 0.1 × SSC and 0.1% SDS. More preferred stringent conditions may be, for example, conditions of using, as a hybridization solution, 50% formamide and 5 x SSC (0.15 M NaCl, 15 mM sodium citrate, pH 7.0), 1 × Denhardt solution, 1% SDS, 10% dextran sulfate, 10 µg/ml denatured salmon sperm DNA, 50 mM phosphate buffer (pH 7.5)).

In the present invention, there may be used a nucleic acid (a nucleic acid fragment) having a part of the nucleotide sequence of the gene of the present invention that encodes the present enzyme or a nucleotide sequence complementary thereto. Such a nucleic acid fragment can be used to, for example, detect, identify, and/or amplify a nucleic acid having the nucleotide sequence of the gene of the present invention that encodes the present enzyme, etc. For example, the nucleic acid fragment is designed to, at least, include a portion that hybridizes to a continuous nucleotide portion (for example, a length consisting of about 10 to about 100 nucleotides, preferably about 20 to about 100 nucleotides, and more preferably about 30 to about 100 nucleotides) in the nucleotide sequence of the gene of the present invention that encodes the present enzyme. When the nucleic acid fragment is utilized as a probe, it can be labeled. For labeling, for example, a fluorescent substance, an enzyme, or a radioisotope can be used.

In the present invention, there may be used recombinant DNA including the above-described gene (i.e. the gene encoding the present enzyme). The recombinant DNA is provided in the form of, for example, a vector. The term "vector" is used in the present description to mean a nucleic acid molecule capable of transporting a nucleic acid inserted in the nucleic acid molecule into a target such as a cell.

Depending on intended purpose (cloning or the expression of a protein), or taking into consideration of the type of a host cell, a suitable vector is selected. Examples of the vector having *Escherichia coli* as a host may include M13 phage or a modified body thereof, λ phage or a modified body thereof, and pBR322 or a modified body thereof (pB325, pAT153, pUC8, etc.). Examples of the vector having yeast as a host may include pYepSec1, pMFa, and pYES2. Examples of the vector having an insect cell as a host may include pAc and pVL. Examples of the vector having a mammalian cell as a host may include pCDM8 and pMT2PC.

The vector is preferably an expression vector. The term "expression vector" means a vector that is capable of introducing a nucleic acid inserted in the vector into a cell of interest (a host cell) and that is also capable of allowing the nucleic acid to express in the host cell. The expression vector usually includes a promoter sequence necessary for the expression of the inserted nucleic acid, an enhancer sequence for promoting the expression, and the like. An expression vector including a selective marker can also be used. In the case of using such an expression vector, the presence or absence of the introduction of the expression vector (and the degree thereof) can be confirmed by utilizing the selective marker.

Insertion of the nucleic acid into the vector, insertion of a selective marker (if necessary), insertion of a promoter (if necessary), and the like can be carried out by using a standard recombinant DNA technique (for example, a publicly known method using restriction enzyme and DNA ligase, which can refer to Molecular Cloning, Third Edition, 1.84, Cold Spring Harbor Laboratory Press, New York).

In terms of the ease of handling, microorganisms such as *Escherichia coli, Bacillus subtilis,* and budding yeast (*Saccharomyces cerevisiae*) are preferably used as host cells. However, any host cells can be utilized, as long as they are capable of replicating recombinant DNA and expressing the gene of the present enzyme. In the case of utilizing a T7 promoter, the used *Escherichia coli* may be, for example, the *Escherichia coli* BL21(DE3)pLysS. In the case of not using a T7 promoter, the used *Escherichia coli* may be, for example, the *Escherichia coli* JM109. In addition, examples of the budding yeast may include the budding yeast SHY2, the budding yeast AH22, and the budding yeast INVSc1 (Invitrogen).

In the present invention, a microorganism including the recombinant DNA (i.e. a transformant) may be used. The microorganism can be obtained by transfection or transformation using the above-described vector. Such transfection or transformation can be carried out, for example, by a calcium chloride method (Journal of Molecular Biology (J. Mol. Biol.), Vol. 53, p. 159 (1970)), a Hanahan method (J. Mol. Biol., Vol. 166, p. 557 (1983)), an SEM method (Gene, Vol. 96, p. 23 (1990)), a method of Chung, et al. (Proceedings of the National Academy of Sciences of the USA. Vol. 86, p. 2172 (1989)), a calcium phosphate coprecipitation method, electroporation (Potter, H. et al., Proc. Natl. Acad. Sci. U.S.A. 81, 7161-7165 (1984)), and lipofection (Felgner, P. L. et al., Proc. Natl. Acad. Sci. U.S.A. 84, 7413-7417 (1984)). Besides, the above-described microorganism can be utilized to produce the enzyme of the present invention.

### 3. Intended uses of the present enzyme agent

A further aspect of the present invention relates to the intended uses of the present enzyme agent. As a first intended use, provided is a method for producing a collagen tripeptide (CTP) (hereinafter referred to as a "CTP production method"). According to the CTP production method of the present invention, the present enzyme agent is allowed to act on collagen or gelatin (denatured collagen). For example, the present enzyme agent is added to a solution of collagen or gelatin, and a reaction is then carried out under conditions of, for example, 20°C to 50°C, and preferably 30°C to 40°C, for a predetermined period of time (for example, 1 hour to 48 hours, preferably 1 hour to 24 hours, and more preferably 1 hour to 12 hours). As a result of a decomposition reaction by collagenase as an active ingredient of the present enzyme agent, a collagen tripeptide is produced. The composition, ratio, etc. of CTP in the product can be fluctuated depending on the type, origin, etc. of the used substrate (collagen or gelatin). According to the production method of the present invention, a composition containing a tripeptide having Gly at the N-terminus thereof (for example, Gly-Pro-Hyp, Gly-Pro-Ala, or Gly-Glu-Arg), namely, a CTP-containing composition can be obtained. CTP can be produced by the single use of the present enzyme agent, and this is one of the characteristics of the present invention. However, it is also possible to use the present enzyme agent in combination with another collagenase or protease, or with peptidase, so as to achieve the improvement of production efficiency, etc.

The origin of the used collagen/gelatin is not particularly limited, and examples of the origin of the used collagen/gelatin may include fish, a pig, a bovine, and a chicken. Commercially available collagen or gelatin may also be used. The method of preparing such collagen or gelatin is not particularly limited, either. For example, a raw material (the skin, bone, tendon of animals, fish scales, etc.) is washed with water and is then dried, and thereafter, the resultant is subjected to a decalcification treatment using hydrochloric acid or the like, as necessary. After washing, the resultant is treated with caustic soda, hydrochloric acid, etc. to obtain crude collagen. In addition, the crude collagen is subjected to a thermal treatment, so that gelatin can be extracted.

After completion of an enzymatic reaction using the present enzyme agent, a purification treatment (for example, filtration, ion exchange, or activated carbon treatment) is carried out, as necessary, for the removal of insoluble components, the improvement of purity, discoloration, deodorization, etc.

A second intended use of the present enzyme agent is the quality improvement of edible meats (tenderization of edible meats, etc.), and is preferably the quality improvement of edible meats containing cartilages, or degradation of cartilages. When the enzyme agent of the present invention is used, cartilages can be efficiently degraded. In the case of edible meats containing cartilages, food texture can be improved. In addition, in the case of edible meats containing bones and cartilages, the cartilages can be easily separated from the bones. The edible meats, with which the enzyme is allowed to react, are not particularly limited, as long as they are edible meats containing cartilages. The term "edible meat" is used herein to include edible meat-processed products. When edible meats containing cartilages are treated with the present enzyme agent, the present enzyme agent may be allowed to act on the edible meats according to a method of immersing edible meats in an enzyme solution (a solution containing the enzyme agent), a method of infiltrating an enzyme solution into edible meats by a pressure treatment, a method of injecting an enzyme solution into edible meats, a method of injecting an enzyme solution into edible meats and then tumbling the meats (a treatment of mechanically infiltrating an enzyme solution into edible meats), or other methods. The temperature conditions applied when the enzyme agent is allowed to act on edible meats is, for example, 4°C to 40°C, and the temperature conditions may also be 4°C to 30°C, 4°C to 25°C, or 4°C to 20°C, or may further be 10°C to 40°C, 20°C to 40°C, 30°C to 40°C, or 35°C to 40°C. The time required for allowing the enzyme agent to act on edible meats (i.e. the reaction time) is, for example, 1 hour to 1 day, and may also be 1 hour to 12 hours, 1 hour to 6 hours, 1 hour to 3 hours, or 1 hour to 2 hour.

### Examples

### < Reference Examples >

### 1. Screening for collagenase-producing strain

In order to find out collagenase that can be utilized for food products, a first screening was carried out using collagenase activity as an indicator from the library of Amano Enzyme Inc., and 113 types of production strains with biosafety level 1 (BSL1), which exhibited high activity, were selected.

Subsequently, as a second screening, the culture supernatant of each of the selected 113 types of production strains was allowed to react with gelatin, and the content rate of peptides having Gly at the N-terminus thereof in the reaction product was then evaluated. As a result, 19 types of production strains were selected. The total peptide amount was quantified using a ninhydrin reagent. On the other hand, the amount of the peptides having Gly at the N-terminus thereof was quantified using Collagen Quantification Kit (manufactured by COSMO BIO COMPANY, LIMITED).

As a third screening, tripeptides were partially purified from the reaction products of the culture supernatants of the 19 types of production strains with gelatin according to gel filtration chromatography, and were then analyzed according to reverse phase chromatography. The culture supernatants of the production strains that seemed to be promising from the analysis results were partially purified, and thereafter, the CTP-producing ability of collagenase was evaluated, so that the *Lysinibacillus fusiformis* 57413 strain was finally selected as a collagenase-producing bacterium.

### 2. Preparation and purification of collagenase crude enzyme solution of 57413 strain

The 57413 strain was subjected to an aerated and agitated culture in a gelatin-containing medium (5% fish gelatin, 0.5% yeast extract, and 2% NaCl) at 30°C for 2 days. Thereafter, the obtained culture solution was centrifuged, and the supernatant was then filtrated using diatomaceous earth to obtain a crude enzyme solution. Thereafter, the enzyme was purified using hydrophobic chromatography (Pheny HP, manufactured by GE Healthcare Life Sciences) and anion exchange chromatography (DEAE FF, manufactured by GE Healthcare Life Sciences ).

### 3. Confirmation of gene sequence of collagenase from 57413 strain

The 57413 strain was cultured in an SCD liquid medium overnight at 30°C, and the cell mass was then recovered by centrifugation. The recovered cell mass was suspended in a TE buffer, and DNA was then extracted using NucleoSpin (registered trademark) Microbial DNA (manufactured by Takara Bio, Inc.). Using the extracted DNA as a template, PCR was carried out employing the following upstream and downstream primers and PrimeSTAR (registered trademark) Max DNA Polymerase (manufactured by Takara Bio, Inc.). The amplified PCR product was subjected to a nucleotide sequence analysis using primers having homology with the inside and outside of the structural gene, so that the sequence of the PCR product was confirmed.

The amino acid sequence as set forth in SEQ ID NO: 5 is the amino acid sequence (mature body) of the collagenase derived from the *Lysinibacillus fusiformis* 57413 strain. Besides, the amino acid sequence of the collagenase derived from the *Lysinibacillus fusiformis* 57413 strain, which also has a signal peptide and a pro-sequence, is shown in SEQ ID NO: 6.

The DNA of SEQ ID NO: 7 encodes only the amino acid sequence of the mature body (SEQ ID NO: 5), whereas the DNA of SEQ ID NO: 8 encodes a signal peptide and a pro-sequence, in addition to the mature body (the amino acid sequence as set forth in SEQ ID NO: 5).

Upstream: forward primer: GGAAACAATCTAAATGTGTCT (SEQ ID NO: 9)
Downstream: reverse primer: CCGCCTTTAAAGGCTCTCCGA (SEQ ID NO: 10)

### 4. Recombinant expression of collagenase

The 57413 strain collagenase gene was introduced into pColdIII (manufactured by Takara Bio, Inc.) to construct an expression plasmid. Using the constructed expression plasmid, the *Escherichia coli* BL21 was transformed according to an ordinary method. The obtained transformant was cultured in an LB medium (supplemented with ampicillin) overnight at 37°C, and the obtained culture solution was then inoculated in a volume of 1% into an LB medium (supplemented with ampicillin). The obtained mixture was cultured at 37°C for 2 hours, followed by addition of IPTG, and the thus obtained mixture was then cultured at 15°C overnight. Thereafter, a cell mass was recovered from the obtained culture solution by centrifugation, and the cell mass was then disintegrated using an ultrasonic integrator. The supernatant was recovered by centrifugation, and was defined as a recombinant crude enzyme solution. The enzyme activity of this crude enzyme solution was confirmed by the following measurement methods. As a result, it was found that the crude enzyme solution had collagen-decomposing activity, pz-peptide-decomposing activity, and CTP-producing ability. Moreover, the crude enzyme solution does not have casein-decomposing activity and it is highly likely that the crude enzyme specifically act on collagen or gelatin.

### < Pz-peptide-decomposing activity >

The enzyme solution (100 µL) was added to 900 µL of 200 mM Tris-HCl buffer containing 1 mg/mL Pz-peptide (Pz-Pro-Leu-Gly-Pro-D-Arg-OH, manufactured by BACHEM) and 20 mM CaCl₂, and a reaction was then initiated at 37°C. Ten and twenty minutes after initiation of the reaction, 100 µL of the reaction solution was sampled, and was then added to 200 µL of 25 mM citric acid solution to prepare a reaction stop solution. To this reaction stop solution, 1 mL of ethyl acetate was added, and then, the mixed solution was stirred for 10 seconds and was then centrifuged (12000 x g, 10 minutes), so that an ethyl acetate layer was recovered as a supernatant. The absorbance of the recovered ethyl acetate layer as a supernatant at 320 nm was measured, so that the amount of Pz-Pro-Leu released by collagenase was obtained. The enzyme activity was evaluated by calculating the rate of producing Pz-Pro-Leu per minute from the amounts of Pz-Pro-Leu produced 10 minutes and 20 minutes later. The amount of enzyme that decomposes 1 µmοl Pz peptide (releases 1 µmοl Pz-Pro-Leu) per minute was defined as 1 U.

### < Collagenase activity >

Collagenase activity was measured using PROTAZYME OL TABLETS (manufactured by Megazyme). A substrate solution (300 µL) prepared by suspending 1 OL tablet in a 200 mM Tris buffer containing 10 mM CaCl₂ was dispensed in a 1.5 mL tube, while stirring, and it was then placed on ice. Into the dispensed substrate solution, 100 µL of the enzyme solution was added and mixed, and the mixed solution was then stirred using BioShaker set at 40°C for 30 minutes, so as to perform a reaction. Thirty minutes after the reaction, 1 mL of 2% trisodium phosphate solution was added to the reaction mixture to terminate the enzyme reaction, and the reaction mixture was then centrifuged (13000 g, 10 minutes). The supernatant (200 µL) was transferred into a microtiter plate, and the absorbance at 590 nm was then measured. The intensity of the collagenase activity was determined based on the value at 590 nm that was increased for 30 minutes.

### < Casein-decomposing activity >

Casein-decomposing activity was measured using PROTAZYME AK TABLETS (manufactured by Megazyme). A substrate solution (300 µL) prepared by suspending 1 AK tablet in a 200 mM Tris buffer containing 10 mM CaCl₂ was dispensed in a 1.5 mL tube, while stirring, and it was then placed on ice. Into the dispensed substrate solution, 100 µL of the enzyme solution was added and mixed, and the mixed solution was then stirred using BioShaker set at 40°C for 30 minutes, so as to perform a reaction. Thirty minutes after the reaction, 1 mL of 2% trisodium phosphate solution was added to the reaction mixture to terminate the enzyme reaction, and the reaction mixture was then centrifuged (13000 g, 10 minutes). The supernatant (200 µL) was transferred into a microtiter plate, and the absorbance at 590 nm was then measured. The intensity of casein-decomposing activity was determined based on the value at 590 nm that was increased for 30 minutes.

### < Confirmation of CTP-producing ability >

A serially diluted enzyme solution was allowed to react with gelatin (gelatin with a final concentration of 2%) for 12 hours, and thereafter, the reaction mixture was boiled for 10 minutes to terminate the reaction. This reaction stop solution was 10-fold diluted with ultrapure water, and was then subjected to gel filtration analysis using Superdex peptide 7.5/300, so that the amount of CTP produced was confirmed. Conditions for the gel filtration were as follows.
Superdex_peptide 7.5/300
Buffer: 0.02 M Phosphate buffer containing 0.25 M NaCl, pH 7
Flow rate: 0.28 mL/min
Applied amount: 100 µL
Detection: 214 nm
System: AKTA/low-temperature storage

### < Examples >

### 1. Preparation of enzyme powders of 7128 strain collagenase

The collagenase-producing bacterium *Lysinibacillus fusiformis* 7128 strain was selected according to the section "1. Screening for collagenase-producing strain" in Reference Examples.

20 L of a gelatin-containing medium (5% fish gelatin, 0.5% yeast extract, and 2% NaCl) was prepared in ajar fermenter (30 L volume), and was then sterilized by steam. To the resulting medium, 200 mL of the 7128 strain cultured in an SCD medium overnight was added, and the obtained mixture was then cultured at 30°C at a stirring number of 200 rpm at an airflow amount of 0.5 vvm for 36 hours. The obtained culture solution was subjected to diatomaceous earth filtration to obtain a crude enzyme solution. This crude enzyme solution was filtrated through 1% activated carbon to obtain a clear filtrate. This clear filtrate was subjected to ultrafiltration (fractional molecular weight: 6000), and was then desalted and concentrated by adding 4 times the volume of water. The resultant was subjected to diatomaceous earth filtration and membrane filtration (0.45 µm), and the thus obtained filtrate was then freeze-dried to obtain enzyme powders of the 7128 strain collagenase.

### 2. Identification of sequences of 7128 strain collagenase

The gene sequence of the 7128 strain collagenase was confirmed using primers designed based on the sequence information of the 57413 stain-derived collagenase. The amino acid sequence thereof was also determined from the gene sequence.

The amino acid sequence as set forth in SEQ ID NO: 1 is the amino acid sequence (mature body) of the collagenase derived from the *Lysinibacillus fusiformis* 7128 strain. Besides, the amino acid sequence of the collagenase derived from the *Lysinibacillus fusiformis* 7128 strain, which also has a signal peptide and a pro-sequence, is shown in SEQ ID NO: 2.

The DNA of SEQ ID NO: 3 encodes only the amino acid sequence of the mature body (SEQ ID NO: 1), whereas the DNA of SEQ ID NO: 4 encodes a signal peptide and a pro-sequence, in addition to the mature body (the amino acid sequence as set forth in SEQ ID NO: 1).

### < SEQ ID NO: 1 >

AEKTPYNVLQMKPIGIEMSKDEMVHSTMAEETLSYEERLEMGDFSQRPAPIMEQMDTQQLQEQYSMAELNNMSN
RELIHTLGSIRWYQITDLFQFNDDTKAFYQNEERMQVIINELGNRGSSFTKDDSKGIETFVEVLRSAFYVAFYN
NELGYLNERSFQDKCLPALNEIAKNPNFKLGTDEQDKVVSAYGKLIGNASSDVETVQHATNILKQYNENLSTYE
SENSKGQAIYDIIHGIDYDLQSYLYDTRKEANTTMWYGKIDSFIEEVNNIALIHNVTDSNSWLINNGIYYAGRL
GQFHSNPNKGLEVVSQAMHMYPYLSEAYFVAVEQITTNYGGRDLNGNTVDLQKVREEGKKQYLPKTYTFDDGSI
VFKTGDQVTEDKIQRLYWAAKEVKAQYHRVIGNDQALEPGNADDILTVVIYNSPDQYRLNRQLYGYETNNGGIY
IEETGTFFTYERTPEQSIYSLEELFRHEYTHFLQGRFEVPGLFGTGDMYQNERLTWFQEGNAEFFAGSTRTNDV
VPRKSIISGLSHDPSQRYTAEQTMFATYGSWDFYNYSFALQSYMYTHQFDMFDRIQDLIRANDVKNYDAYRDTL
SKDSQLNMEYQAYMQQLIDNQETYEVPQVAEDYLMPHEPKALNEVQQEIVDIAHVKDANMTKHQSQFFNTFTLE
GTYVGSATQGESQDWKTMSKQVNQMLEQLSQKEWSGYKTMTAYFVNYRVNAANQFEYDVVFHGVSTDDGETQAP
IVQVNGPYTGMINEKIQFNSDGSKDTDGEIVSYLWDFGDGATSEAANPTHVYENEGTYKVTLTVKNNKGQESKG
QTTATVQKGGQTGQEHAMTIPFNKPIKGSLIENDTNVYQFDITSLEEIDISVVNENQIGMTWVLYHESDKENYV
AYGQEDGQTIKGKYNAKPGKYYLYVYKFDDENGTYTVQVQNSTKTEIEPNNRPEEATMLPFHTPLSGSLMEDDH
TDVYEFNVTSPKEIDISVLNENQIGMTWVLYHESDSQNYASFGQEDGNMINGKLNAKPGKYYLYVYKFENENGT

YTVHVQ

### 3. Enzymatic properties of 7128 strain collagenase

### (1) Optimal temperature

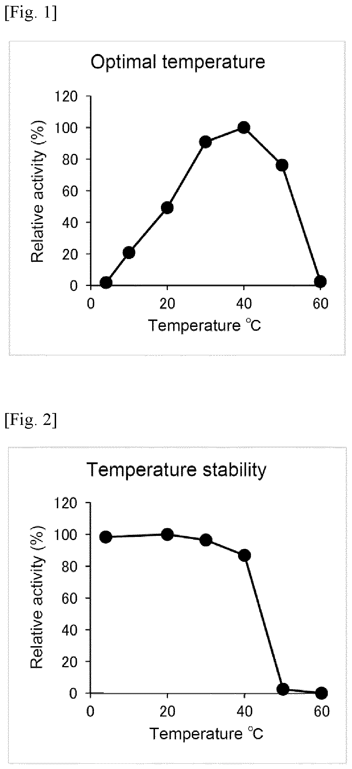

The influence of the temperature on the reactivity of the present enzyme was confirmed. A measurement method using Pz-peptide as a substrate was applied, and the activity was measured, while the temperature upon the reaction was changed from 4°C to 60°C. The optimal temperature was evaluated using relative activity obtained when the maximum activity (the highest value of the activity) was set at 100%. As shown in Fig. 1, the optimal temperature was around 40°C.

### (2) Temperature stability

The temperature stability of the present enzyme was examined. A sample prepared by 5-fold diluting the present enzyme solution with a 200 mM PIPES buffer (pH 7.0) was treated at each temperature (4°C, 20°C, 30°C, 40°C, 50°C, and 60°C) for 30 minutes, and the activity was then measured by a measurement method using Pz-peptide as a substrate. The temperature stability was evaluated using relative residual activity to the activity at a treatment temperature with the highest activity as 100%. As shown in Fig. 2, it was found that the activity was not decreased from the treatment at 4°C to the treatment at 40°C, and thus that the activity was stable until 40°C.

### (3) Optimal pH

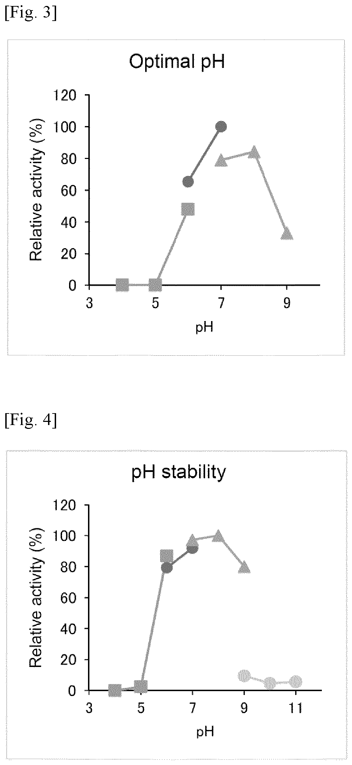

The influence of pH on the reactivity of the present enzyme was examined. As a buffer for dissolving Pz-peptide, each 200 mM buffer (i.e. an acetate buffer at pH 4, 5 or 6; a PIPES buffer at pH 6 or 7; a Tris-HCl buffer at pH 7, 8 or 9; and a glycine buffer at pH 9, 10 or 11) was used, and the activity was measured. The optimal pH was evaluated using relative activity obtained when the maximum activity was set at 100%. As shown in Fig. 3, the optimal pH was found to be around pH 7.

### (4) pH Stability

The pH stability of the present enzyme was examined. The present enzyme solution was 5-fold diluted with each 200 mM buffer (in which an acetate buffer was used at pH 4, 5 or 6; a PIPES buffer was used at pH 6 or 7; a Tris-HCl buffer was used at pH 7, 8 or 9; and a glycine buffer was used at pH 9, 10 or 11), and the diluted solution was then treated at 30°C for 60 minutes. Thereafter, the activity was measured by a measurement method using Pz-peptide as a substrate. The pH stability was evaluated using relative residual activity to the activity at the treatment pH with the highest activity as 100%. As shown in Fig. 4, it was found that high activity (80% or more) was maintained in the pH range from about 6 to about 9 (Tris-HCl), and that the enzyme was stable in this pH range.

### 4. Confirmation of CTP-producing ability of 7128 strain collagenase

The 7128 strain collagenase (the enzyme of the present invention), the 57413 strain collagenase, and Streptomyces-derived collagenase for comparison use were used to confirm the production of a collagen tripeptide.

A serially diluted enzyme solution was allowed to react with fish gelatin with a final concentration of 2% or beef bone gelatin with a final concentration of 2% at 40°C for 12 hours, and the reaction product was then boiled for 10 minutes to terminate the reaction. This reaction stop solution was 10-fold diluted with ultrapure water, and was then subjected to gel filtration analysis using Superdex peptide 7.5/300, so as to confirm the amount of CTP produced. The conditions for gel filtration were as follows.

Superdex_peptide 7.5/300
Buffer: 0.02 M Phosphate buffer containing 0.25 M NaCl, pH 7
Flow rate: 0.28 mL/min
Applied amount: 100 µL
Detection: 214 nm
System: AKTA/low-temperature storage

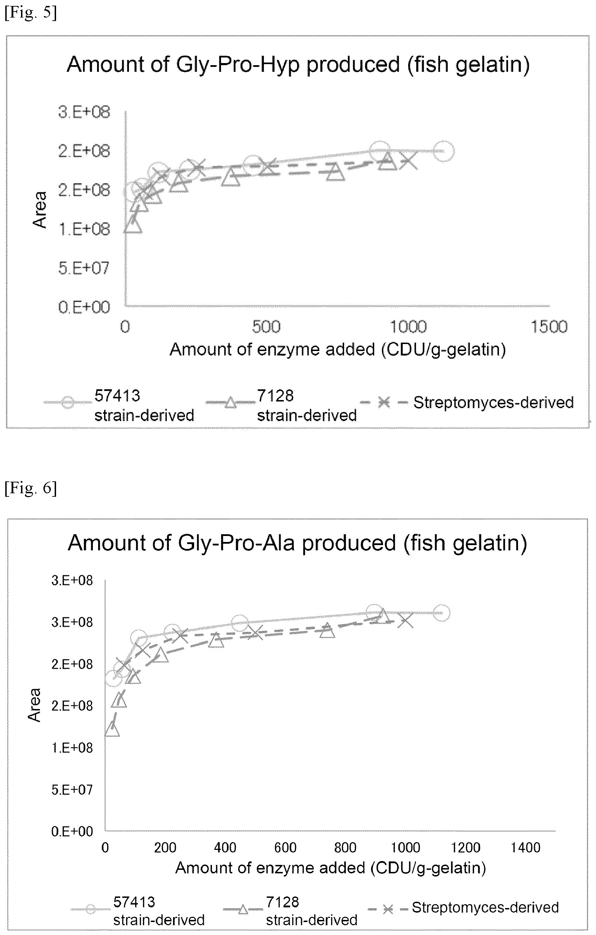

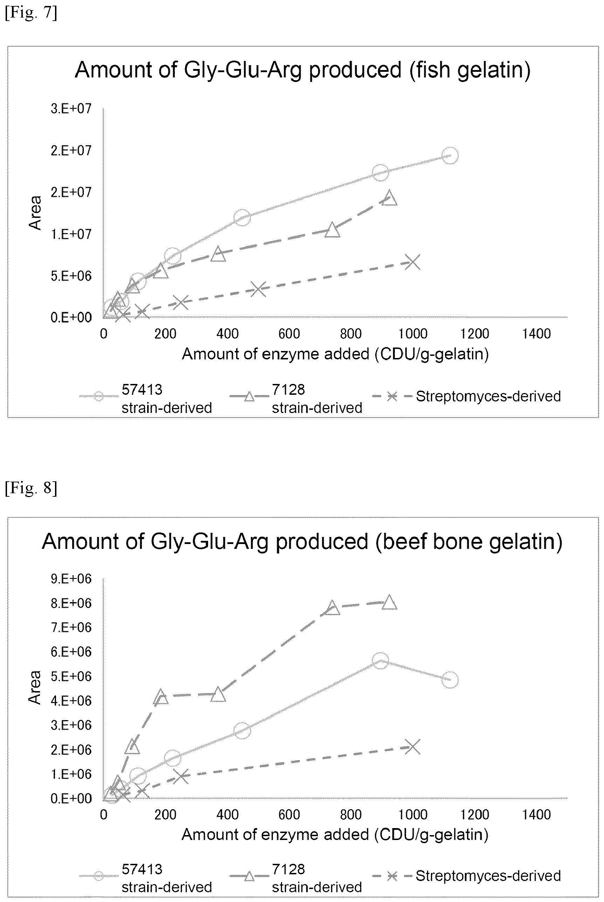

The results are shown in Fig. 5 to Fig. 8.

It was found that the enzyme of the present invention exhibits an ability to produce Gly-Pro- Hyp, Gly-Pro-Ala, and Gly-Glu-Arg. In particular, it was found that the enzyme of the present invention has an excellent ability to produce Gly-Glu-Arg.

### 5. Chicken cartilage degradation ability of 7128 strain collagenase

To 0.44 g of chicken breast cartilage cut into 5 mm cubes, 5 mL of 8 mg/mL enzyme solution was added, followed by performing a shaking treatment at 37°C for 1.5 hours. The treated sample was filtrated through gauze to collect the residue. The residue was washed with purified water, and was then left at room temperature overnight to dry it. Thereafter, the weight of the dried residue was measured. As a blank, the operations were carried out in the same manner as that described above, using purified water instead of the enzyme solution. As a comparison, the operations were carried out in the same manner as that described above, using the 57413 strain-derived collagenase and a commercial meat tenderizer "SUJIMADE YAWARAKA SYOKUNIN" (KITII CORPORATION). The cartilage degradation degree was calculated according to the following equation.Cartilage degradation degree (%) = (weight of dry residue of blank - weight of enzyme-treated dry residue) / weight of dry residue of blank x 100

### < Results >

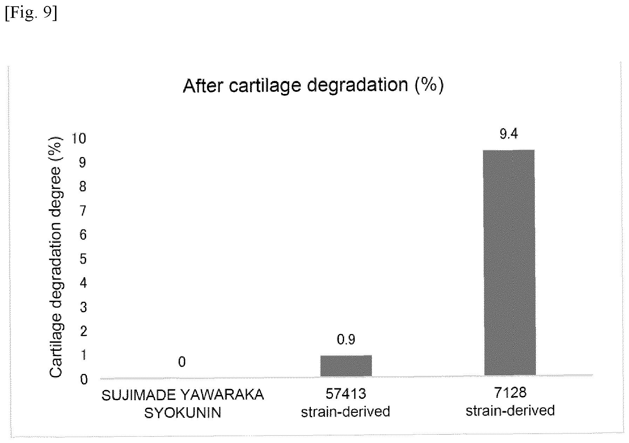

The results are shown in Fig. 9.

It was demonstrated that the 7128 strain-derived collagenase of the present invention has an excellent cartilage degradation ability.

### Industrial Applicability

The enzyme agent of the present invention includes, as an active ingredient, a collagenase derived from highly safe microorganisms. Accordingly, the enzyme agent of the present invention is suitable for the use in the field of food products or medical purposes, and thus, the present enzyme agent is industrially highly valuable.

The present invention is not limited to the above-described embodiments and examples of the invention in any way. The present invention includes various modified aspects that can be easily conceived of by a person skilled in the art without departing from the scope of claims. The contents of the study papers, published patent publications, patent publications, and the like that are explicitly specified in the present description shall be cited by incorporating the entire contents thereof.

### [Sequence Listing Free Text]

SEQ ID NO: 9: explanation of artificial sequence: forward primer
SEQ ID NO: 10: explanation of artificial sequence: reverse primer

## Claims

1. An enzyme agent for collagen tripeptide production, comprising, as an active ingredient, a collagenase consisting of the amino acid sequence as set forth in SEQ ID NO: 1 or an amino acid sequence equivalent thereto.

2. The enzyme agent according to claim 1, wherein the collagenase consisting of the equivalent amino acid sequence has an identity of 90% or more to the amino acid sequence as set forth in SEQ ID NO: 1, and has an ability to produce Gly-Glu-Arg from beef bone gelatin that is equivalent to that of the collagenase consisting of the amino acid sequence as set forth in SEQ ID NO: 1.

3. The enzyme agent according to claim 1 or 2, wherein the collagenase is derived from *Lysinibacillus fusiformis.*

4. An enzyme agent for collagen tripeptide production, which is derived from *Lysinibacillus fusiformis* and comprises, as an active ingredient, a collagenase having the following physicochemical properties:
(1) optimal temperature: around 40°C;
(2) temperature stability: relative residual activity to the activity at a treatment temperature with the highest activity as 100% is 80% or more from 4°C to 40°C, and is 10% or less at 50°C;
(3) optimal pH: around pH 7; and
(4) pH stability: relative residual activity to the activity at the treatment pH with the highest activity as 100% is 80% or more in the pH range of 6 to 8, and is 10% or less at pH 5.

5. An enzyme agent for edible meat tenderization and/or cartilage degradation, comprising, as an active ingredient, a collagenase consisting of the amino acid sequence as set forth in SEQ ID NO: 1 or an amino acid sequence equivalent thereto.

6. The enzyme agent according to claim 5, wherein the collagenase consisting of the equivalent amino acid sequence has an identity of 90% or more to the amino acid sequence as set forth in SEQ ID NO: 1, and has a chicken cartilage degradation ability equivalent to that of the collagenase consisting of the amino acid sequence as set forth in SEQ ID NO: 1.

7. The enzyme agent according to claim 5 or 6, wherein the collagenase is derived from *Lysinibacillus fusiformis.*

8. An enzyme agent for edible meat tenderization and/or cartilage degradation, which is derived from *Lysinibacillus fusiformis* and comprises, as an active ingredient, a collagenase having the following physicochemical properties:
(1) optimal temperature: around 40°C;
(2) temperature stability: relative residual activity to the activity at the treatment temperature with the highest activity as 100% is 80% or more from 4°C to 40°C, and is 10% or less at 50°C;
(3) optimal pH: around pH 7; and
(4) pH stability: relative residual activity to the activity at the treatment pH with the highest activity as 100% is 80% or more in the pH range of 6 to 8, and is 10% or less at pH 5.

9. A method for producing a collagen tripeptide, which is **characterized in that** it comprises allowing the enzyme agent according to any one of claims 1 to 4 to act on collagen or gelatin.

10. A method for edible meat tenderization and/or cartilage degradation, which is **characterized in that** it comprises allowing the enzyme agent according to any one of claims 5 to 8 to act on edible meat and/or cartilage.
